# EUROPEAN PATENT APPLICATION

(11) **EP 4 537 844 A1**
(43) Date of publication of application: **16.04.2025**
(21) Application number: 23819919.4
(22) Date of filing: 09.06.2023
(51) Int. Cl.: A61K 45/00, A61K 31/40, A61P 35/00, C12Q 1/02, G01N 33/15

(54) **PROPHYLACTIC OR THERAPEUTIC AGAINST EXACERBATION OR RECURRENCE OF CANCER, AND SCREENING METHOD THEREFOR**

(30) Priority: 09.06.2022 US 202263350414 P
(71) Applicant: Keio University, Tokyo, 108-8345 (JP)
(72) Inventor: SATO Toshiro, Tokyo 160-8582 (JP); OHTA Yuki, Tokyo 160-8582 (JP); TAKANO Ai, Tokyo 160-8582 (JP)
(74) Representative: Cabinet Laurent & Charras
(86) International application number: PCT/JP2023/021569
(87) International publication number: WO 2023/238939

(57) **Abstract**

What are provided are: a prophylactic or therapeutic agent against relapse or recurrence of cancer due to cells including dormant cancer stem cells during chemotherapy, the prophylactic or therapeutic agent including a YAP inhibitor as an active component; and a screening method including a step (a) of allowing a chemotherapeutic agent to act on cancer organoids, resulting in death of most of the cancer organoids, a step (b) of further incubating the cancer organoids, resulting in start of proliferation of cells including dormant cancer stem cells present in the cancer organoids, and a step (c) of evaluating the proliferation of the cells including the dormant cancer stem cells, in which the step (a) or (b) is carried out in the presence of a test substance, and the fact that the proliferation of the cells including the dormant cancer stem cells is inhibited compared to that in the absence of the test substance indicates that the test substance is a prophylactic or therapeutic agent against relapse or recurrence of cancer due to cells including dormant cancer stem cells during chemotherapy.

## Description

### TECHNICAL FIELD

The present invention relates to a prophylactic or therapeutic agent against relapse or recurrence of cancer, and a screening method therefore. Priority is claimed on United States Patent No. 63/350,414, provisionally filed in the United States June 9, 2022, the content of which is incorporated herein by reference.

### BACKGROUND ART

The number of colorectal cancer patients in Japan ranks first among all cancers, and the number of deaths in 2020 ranks first among women and third among men, casting a heavy shadow on society. Recent advances in chemotherapy have improved treatment results for unresectable advanced cancer, but the number of deaths from colorectal cancer still remains high.

Incidentally, Patent Document 1 describes a method for screening anticancer agents effective for cancer stem cells by targeting LGR5-positive cancer stem cells. In addition, it is described that even if LGR5-positive cancer stem cells are killed, the tumor size eventually increases again, LGR5-negative cancer cells have the ability to dedifferentiate into LGR5-positive cancer stem cells, and combined use of cancer stem cell-specific anticancer agents and existing anticancer agents is effective in cancer treatment.

In addition, Non Patent Document 1 describes that mRNA and protein levels of COL17A1 (collagen type XVIIα1 chain) are elevated in pancreatic adenocarcinoma (PDAC), that PDAC patients with a high COL17A1 expression have a lower overall survival rate than those with a low COL17A1 expression, that COL17A1 knockdown significantly inhibited tumor proliferation and invasion in PDAC cells, and that cells with COL17A1 overexpression had a significantly higher proliferative and invasive capacity.

### Citation List

### Patent Document

Patent Document 1: PCT International Publication No. WO2018/181276

### Non Patent Document

Non Patent Document 1: Mao F., et al., High Expression of COL17A1 Predicts Poor Prognosis and Promotes the Tumor Progression via NF-kappaB Pathway in Pancreatic Adenocarcinoma, Journal of Oncology, Article ID 8868245, 2020.

### SUMMARY OF INVENTION

### Technical Problem

Chemotherapy for cancer has problems of relapse or recurrence due to the presence of cancer cells that are resistant to chemotherapeutic agents. An object of the present invention is to elucidate the mechanism of relapse or recurrence of cancer after chemotherapy and to provide a prophylactic or therapeutic agent against relapse or recurrence of cancer after chemotherapy, and a screening method therefor.

### Solution to Problem

The present invention has the following aspects.
[1] A prophylactic or therapeutic agent against relapse or recurrence of cancer due to cells including dormant cancer stem cells in cancer tissue during chemotherapy, the prophylactic or therapeutic agent including: a YAP inhibitor as an active component.
[2] The prophylactic or therapeutic agent according to [1], in which the dormant cancer stem cells during chemotherapy are LGR5-positive p27-positive cancer stem cells.
[3] The prophylactic or therapeutic agent according to [1] or [2], in which the relapse or recurrence of cancer is relapse or recurrence of cancer after chemotherapy.
[4] The prophylactic or therapeutic agent according to any one of [1] to [3], in which the YAP inhibitor is an inhibitor of YAP signaling activation.
[5] A pharmaceutical composition for preventing or treating relapse or recurrence of cancer due to cells including dormant cancer stem cells in cancer tissue during chemotherapy, the composition including: the prophylactic or therapeutic agent according to any one of [1] to [4]; and a pharmaceutically acceptable carrier.
[6] A method for screening a prophylactic or therapeutic agent against relapse or recurrence of cancer due to cells including dormant cancer stem cells in cancer tissue during chemotherapy, the method including: a step (a) of allowing a chemotherapeutic agent to act on cancer organoids, resulting in death of most cells of the cancer organoids, a step (b) of further incubating the cancer organoids, resulting in start of proliferation of cells including dormant cancer stem cells present in the cancer organoids, and a step (c) of evaluating the proliferation of the cells including the dormant cancer stem cells, in which the step (a) or (b) is carried out in the presence of a test substance, and the fact that the proliferation of the cells including the dormant cancer stem cells is inhibited compared to that in the absence of the test substance indicates that the test substance is a prophylactic or therapeutic agent against relapse or recurrence of cancer due to cells including dormant cancer stem cells in cancer tissue during chemotherapy.
[7] A method for screening a prophylactic or therapeutic agent against relapse or recurrence of cancer due to cells including dormant cancer stem cells in cancer tissue during chemotherapy, the method including: a step (a) of allowing a chemotherapeutic agent to act on cancer organoids, resulting in death of most cells of the cancer organoids; a step (b) of further incubating the cancer organoids, resulting in start of proliferation of cells including dormant cancer stem cells present in the cancer organoids; and a step (c) of evaluating activation of YAP signaling in the cells including the dormant cancer stem cells, in which the step (a) or (b) is carried out in the presence of a test substance, and the fact that the activation of YAP signaling is inhibited compared to that in the absence of the test substance indicates that the test substance is a prophylactic or therapeutic agent against relapse or recurrence of cancer due to cells including dormant cancer stem cells in cancer tissue during chemotherapy.
[8] A method for screening a prophylactic or therapeutic agent against relapse or recurrence of cancer due to cells including dormant cancer stem cells in cancer tissue during chemotherapy, the method including: a step of incubating LGR5-positive p27-positive cancer stem cells dissociated into single cells in the presence of a test substance; and a step of evaluating the proliferation of the LGR5-positive p27-positive cancer stem cells, in which the fact that the proliferation of the LGR5-positive p27-positive cancer stem cells is inhibited compared to that in the absence of the test substance indicates that the test substance is a prophylactic or therapeutic agent against relapse or recurrence of cancer due to cells including dormant cancer stem cells in cancer tissue during chemotherapy.
[9] A method for screening a prophylactic or therapeutic agent against relapse or recurrence of cancer due to cells including dormant cancer stem cells in cancer tissue during chemotherapy, the method including: a step of incubating LGR5-positive p27-positive cancer stem cells dissociated into single cells in the presence of a test substance; and a step of evaluating activation of YAP signaling in the LGR5-positive p27-positive cancer stem cells, in which the fact that the activation of YAP signaling is inhibited compared to that in the absence of the test substance indicates that the test substance is a prophylactic or therapeutic agent against relapse or recurrence of cancer due to cells including dormant cancer stem cells in cancer tissue during chemotherapy.
[10] A method for screening a prophylactic or therapeutic agent against relapse or recurrence of cancer due to cells including dormant cancer stem cells in cancer tissue during chemotherapy, the method including: a step of incubating LGR5-positive p27-positive cancer stem cells dissociated into single cells in the presence of a test substance; and a step of evaluating expression of COL17A1 in the LGR5-positive p27-positive cancer stem cells, in which the fact that the expression of COL17A1 is elevated compared to that in the absence of the test substance indicates that the test substance is a prophylactic or therapeutic agent against relapse or recurrence of cancer due to cells including dormant cancer stem cells in cancer tissue during chemotherapy.
[11] A method for screening a prophylactic or therapeutic agent against relapse or recurrence of cancer due to cells including dormant cancer stem cells in cancer tissue during chemotherapy, the method including: a step of allowing a chemotherapeutic agent to act on cancer organoids in the presence of a test substance; and a step of evaluating expression of COL17A1 in the cancer organoids, in which the fact that the expression of COL17A1 is elevated compared to that in the absence of the test substance indicates that the test substance is a prophylactic or therapeutic agent against relapse or recurrence of cancer due to cells including dormant cancer stem cells in cancer tissue during chemotherapy.

### Advantageous Effects of Invention

According to the present invention, it is possible to provide a prophylactic or therapeutic agent against relapse or recurrence of cancer after chemotherapy, and a screening method therefor.

### BRIEF DESCRIPTION OF DRAWINGS

[FIG. 1] A schematic view illustrating an experimental system of Experimental Example 1.
[FIG. 2] A graph showing results of Experimental Example 2.
[FIG. 3] A graph showing results of measuring the proportion of divided cells over time in Experimental Example 4.
[FIG. 4] Photographs showing results of Experimental Example 4.
[FIG. 5] A graph showing results of Experimental Example 5.
[FIG. 6] A volcano plot showing results obtained by comparing LGR5-positive p27-positive cells with LGR5-positive p27-negative cells in Experimental Example 6.
[FIG. 7] Fluorescence micrographs showing results of Experimental Example 7.
[FIG. 8] A schematic view showing an experimental schedule and a graph showing change in tumor size in mice over time in Experimental Example 8.
[FIG. 9] Micrographs and graphs showing results of Experimental Example 9.
[FIG. 10] Confocal micrographs and graphs showing results of Experimental Example 9.
[FIG. 11] Confocal micrographs and graphs showing results of Experimental Example 10.
[FIG. 12] An image showing results of capillary-based immunoassay in Experimental Example 11.
[FIG. 13] Confocal micrographs showing results of Experimental Example 11.
[FIG. 14] A graph showing results of Experimental Example 11.
[FIG. 15] A schematic view showing an experimental schedule and micrographs showing results of immunostaining in Experimental Example 12.
[FIG. 16] A graph showing change in tumor size in mice over time in Experimental Example 12.
[FIG. 17] A schematic view showing an experimental schedule and a graph showing change in tumor size in mice over time in Experimental Example 13.
[FIG. 18] A schematic view showing an experimental schedule and a graph showing change in tumor size in mice over time in Experimental Example 14.
[FIG. 19] A schematic view showing a mechanism by which dormant LGR5-positive p27-positive cancer stem cells are released from dormancy and tumors regrow, and a schematic view illustrating how COL17A1 maintains the dormant state of cancer stem cells and how FAK-YAP signaling is activated after chemotherapy to release the dormant state.

### DESCRIPTION OF EMBODIMENTS

### [Prophylactic agent against relapse or recurrence of cancer]

In one embodiment, the present invention provides a prophylactic or therapeutic agent against relapse or recurrence of cancer due to cells including dormant cancer stem cells in cancer tissue during chemotherapy, the prophylactic or therapeutic agent containing a YAP inhibitor as an active component.

As will be described below in examples, the present inventors have revealed that the dormant cancer stem cells during chemotherapy are LGR5-positive p27-positive cancer stem cells. In addition, the present inventors have revealed that LGR5-positive p27-positive cancer stem cells resistant to chemotherapy do not undergo cell division under chemotherapy, are dormant, and proliferate after chemotherapy. The dormant state means that cells do not divide and may be in the G0 phase of the cell cycle. The present inventors have revealed that the proliferation of the dormant LGR5-positive p27-positive cancer stem cells after chemotherapy caused relapse or recurrence of cancer after chemotherapy.

In addition, the present inventors have revealed the mechanism of regrowth of cells including the LGR5-positive p27-positive cancer stem cells relating to relapse or recurrence of cancer and have revealed that YAP inhibitors have a preventive effect or a therapeutic effect on relapse or recurrence of cancer in vitro and in vivo. Therefore, according to the present invention, it is possible to provide a prophylactic or therapeutic agent against relapse or recurrence of cancer after chemotherapy. By administering the prophylactic or therapeutic agent of the present embodiment to post-chemotherapy cancer patients, it is possible to prevent or treat relapse or recurrence of cancer after chemotherapy.

In the present specification, relapse of cancer means that cancer cells that have not been completely killed regrow after treatment of cancer. In addition, recurrence of cancer means that cancer that has once been completely cured by treatment of the cancer recurs at the primary site, or that cancer cells metastasize and regrow at sites other than the primary site.

According to the prophylactic or therapeutic agent of the present embodiment, it is possible to prevent or treat relapse or recurrence of cancer after chemotherapy. The prophylactic or therapeutic agent of the present embodiment can also be referred to as, for example, a regrowth inhibitor and/or an inhibitor of relapse or recurrence of cancer after chemotherapy, and a dormancy release inhibitor for cells including dormant cancer stem cells in cancer tissue during chemotherapy.

**In** the prophylactic or therapeutic agent of the present embodiment, examples of cancer include cancer that has been relapsed or recurred after chemotherapy, and more specific examples thereof include colorectal cancer, gastric cancer, esophageal cancer, colon cancer, rectal cancer, pancreatic cancer, breast cancer, ovarian cancer, prostate cancer, renal cell cancer, and liver cancer.

The chemotherapy means a method of treating cancer using a chemotherapeutic agent. The chemotherapeutic agent means a cytotoxic anticancer agent among anticancer agents. Examples of cytotoxic anticancer agents include topoisomerase inhibitors, platinum preparations, antitumor antibiotics, alkylating agents, metabolic antagonists, and microtubule-acting drugs.

Examples of topoisomerase inhibitors include irinotecan, camptothecin, nogitecan, doxorubicin, amrubicin, and etoposide.

Examples of platinum preparations include cisplatin, carboplatin, nedaplatin, and oxaliplatin.

Examples of antitumor antibiotics include actinomycin D, aclarubicin, amrubicin, idarubicin, epirubicin, zinostatin stimalamer, daunorubicin, pirarubicin, bleomycin, peplomycin, mitomycin C, and mitoxantrone.

Examples of alkylating agents include ifosfamide, cyclophosphamide, dacarbazine, temozolomide, nimustine, busulfan, procarbazine, melphalan, and ranimustine.

Examples of metabolic antagonists include pemetrexed, tegafur, gimeracil, oteracil, tegafur-uracil, 5-fluorouracil (5-FU), and gemcitabine.

Examples of microtubule-acting drugs include paclitaxel, docetaxel, vinorelbine, and vindesine.

In the prophylactic of the present embodiment, the YAP inhibitor is not particularly limited as long as it is a substance that inhibit the activation of YAP signaling. Phosphorylation and activation of focal adhesion kinase (FAK) is known to mediate nuclear translocation of Yes-associated protein (YAP) which is a transcriptional coactivator. The nuclear-translocated YAP is known to conjugate with a transcription factor TEAD together with TAZ to activate transcription of genes involved in proliferation or survival of cells.

The activation of YAP signaling in the present specification means phosphorylation of FAK, nuclear translocation of YAP, binding of YAP, TAZ and TEAD, and induction of target gene expression.

Therefore, the YAP inhibitor may be an inhibitor of phosphorylation of FAK, an inhibitor of binding YAP, TAZ and TEAD, or a substance of inhibiting expression of YAP or TAZ.

Examples of inhibitors of phosphorylation of FAK (FAK inhibitors) include PF573228 (CAS No. 869288-64-2), PF-00562271 Besylate (CAS No. 939791-38-5), PF-562271 (CAS No. 717907-75-0), PF-562271 HCl (CAS No. 939791-41-0), PRT062607 (P505-15) HCl (CAS No. 1370261-97-4), TAE226 (NVP-TAE226) (CAS No. 761437-28-9), GSK215 (CAS No. 2743427-26-9), ALK inhibitor 1 (CAS No. 761436-81-1), BI-4464 (CAS No. 1227948-02-8), GSK2256098 (CAS No. 1224887-10-8), PF-431396 (CAS No. 717906-29-1), PND-1186 (VS-4718) (CAS No. 1061353-68-1), Masitinib mesylate (CAS No. 1048007-93-7), AMP-945 (CAS No. 1393653-34-3), Y15 (CAS No. 4506-66-5), Defactinib (VS-6063) (CAS No. 1073154-85-4), and Solanesol (Nonaisoprenol) (CAS No. 13190-97-1).

Examples of inhibitors of binding YAP, TAZ and TEAD include MYF-01-37 (CAS No. 2416417-65-5), K-975 (CAS No. 2563855-03-6), MGH-CP1 (CAS No. 896657-58-2), VT103 (CAS No. 2290608-13-6), and TED-347 (CAS No. 2378626-29-8), which are TEAD inhibitors (TEADi).

Examples of substances of inhibiting expression of YAP or TAZ include siRNA and shRNA against mRNA of YAP or TAZ.

The prophylactic or therapeutic agent of the present embodiment may be formulated as a pharmaceutical composition mixed with a pharmaceutically acceptable carrier. **In** other words, in one embodiment, the present invention provides a pharmaceutical composition for preventing or treating relapse or recurrence of cancer due to cells including dormant cancer stem cells in cancer tissue during chemotherapy, the composition including: the above-described prophylactic or therapeutic agent; and a pharmaceutically acceptable carrier.

The pharmaceutical composition can be administered, for example, orally in the form of tablets, capsules, elixirs, microcapsules, and the like, or parenterally in the form of injections, suppositories, and the like.

As a pharmaceutically acceptable carrier, it is possible to use those normally used in preparations of pharmaceutical compositions. More specific examples thereof include: binders such as hypromellose, dextrin, macrogol 400, gelatin, corn starch, tragacanth gum, gum arabic; excipients such as lactose hydrate, D-mannitol, starch, crystalline cellulose, and alginic acid; injection solvents such as water, ethanol, and glycerol.

The pharmaceutical composition of the present embodiment may contain an additive. Examples of additives include: lubricants such as calcium stearate and magnesium stearate; sweeteners such as sucrose, lactose, saccharin, and maltitol; flavoring agents such as peppermint and akamono oil; stabilizers such as carmellose sodium, hardened oil, light anhydrous silicic acid, povidone, glycerin fatty acid esters, benzyl alcohol, and phenol; buffer agents such as phosphates and sodium acetate; dissolution assistants such as benzyl benzoate and benzyl alcohol; and colorants such as yellow iron sesquioxide, iron sesquioxide, black iron oxide, and titanium oxide.

The pharmaceutical composition can be formulated by appropriately combining the above-described YAP inhibitor with the above-described pharmaceutically acceptable carrier and additive in a unit dosage form required for generally accepted pharmaceutical practice. **In** the pharmaceutical composition, the YAP inhibitor may be used alone or a combination of two or more thereof may be used.

The appropriate daily dosage of the prophylactic, therapeutic, or pharmaceutical composition is an amount containing an active component (YAP inhibitor) in the lowest effective dosage in view of producing a therapeutic effect. The lowest effective dosage depends on various factors including an activity of an active component contained in a pharmaceutical composition, functional group modification that defines liposolubility and water solubility, administration routes, administration time, discharge rate of a specific active component used, treatment periods, other drugs used in combination, age, sex, weight, diseases, health condition, patient's pre-existing condition, and other factors known in the medical art.

**In** general, the dosage of the prophylactic, therapeutic, or pharmaceutical composition is about 0.0001 to 100 mg/kg body weight of an active component (YAP inhibitor) per day. The prophylactic, therapeutic, or pharmaceutical composition may be administered only once a day or administered in two or four divided doses.

### [Method for screening prophylactic or therapeutic agent against relapse or recurrence of cancer]

### (First Embodiment)

**In** one embodiment, the present invention provides a method for screening a prophylactic or therapeutic agent against relapse or recurrence of cancer due to cells including dormant cancer stem cells in cancer tissue during chemotherapy, the method including: a step (a) of allowing a chemotherapeutic agent to act on cancer organoids, resulting in death of most cells of the cancer organoids, a step (b) of further incubating the cancer organoids, resulting in start of proliferation of cells including dormant cancer stem cells present in the cancer organoids, and a step (c) of evaluating the proliferation of the cells including the dormant cancer stem cells, in which the step (a) or (b) is carried out in the presence of a test substance, and the fact that the proliferation of the cells including the dormant cancer stem cells is inhibited compared to that in the absence of the test substance indicates that the test substance is a prophylactic or therapeutic agent against relapse or recurrence of cancer due to cells including dormant cancer stem cells in cancer tissue during chemotherapy.

By the screening method of the present embodiment, it is possible to screen a prophylactic or therapeutic agent against relapse or recurrence of cancer due to cells including dormant cancer stem cells in cancer tissue during chemotherapy. **In** the screening method of the present embodiment, the same as described above is applied to relapse or recurrence of cancer, chemotherapy, a dormant state, and the like. The dormant cancer stem cells under chemotherapy are preferably LGR5-positive p27-positive cells.

In the screening method of the present embodiment, the step (a) or (b) is carried out in the presence of a test substance. That is, a step of allowing a chemotherapeutic agent to act on cancer organoids may be carried out in the presence of a test substance. Alternatively, a step of allowing a chemotherapeutic agent to act on cancer organoids, followed by further incubating the cancer organoids in the absence of the chemotherapeutic agent may be carried out in the presence of a test substance. Alternatively, both a step of allowing a chemotherapeutic agent to act on cancer organoids and a step of allowing a chemotherapeutic agent to act, followed by further incubating cancer organoids may be carried out in the presence of a test substance.

The organoids are small organs formed of cells accumulated, and have a structure and function similar to those in a living body. In recent years, research on production of various organoids from pluripotent stem cells and biological tissues are being actively conducted, and for example, intestinal organoids, liver organoids, kidney organoids, and brain organoids are produced. Examples of cancer organoids include organoids derived from cancer tissue of a patient. Examples of cancer include those described above.

The test substance is not particularly limited, and, for example, a natural compound library, a synthetic compound library, and an existing drug library can be used.

In the evaluation of the proliferation of the cells including the cancer stem cells in the step (c), proliferation of cells derived from cancer stem cells may be evaluated. That is, proliferation of cells including cancer stem cells that maintain stem cell properties and cells differentiated from cancer stem cells may be evaluated.

The evaluation of the proliferation of the cells including the cancer stem cells can be carried out through an appropriate method, and examples thereof include evaluation based on measurement of the number of cells based on microscopic observation, measurement of the number of cells by measuring ATP, and measurement of the number of cells using tetrazolium compounds such as MTT. If the proliferation of organoids has been inhibited compared to that in the absence of the test substance, it can be determined that the test substance is a prophylactic or therapeutic agent against relapse or recurrence of cancer due to cells including dormant cancer stem cells during chemotherapy.

### (Second Embodiment)

In one embodiment, the present invention provides a method for screening a prophylactic or therapeutic agent against relapse or recurrence of cancer due to cells including dormant cancer stem cells in cancer tissue during chemotherapy, the method including: a step (a) of allowing a chemotherapeutic agent to act on cancer organoids, resulting in death of most cells of the cancer organoids; a step (b) of further incubating the cancer organoids, resulting in start of proliferation of cells including dormant cancer stem cells present in the cancer organoids; and a step (c) of evaluating activation of YAP signaling in the cells including the dormant cancer stem cells, in which the step (a) or (b) is carried out in the presence of a test substance, and the fact that the activation of YAP signaling is inhibited compared to that in the absence of the test substance indicates that the test substance is a prophylactic or therapeutic agent against relapse or recurrence of cancer due to cells including dormant cancer stem cells in cancer tissue during chemotherapy.

The screening method of the second embodiment differs mainly from the screening method of the first embodiment in that it evaluates activation of YAP signaling in the cells including the dormant cancer stem cells.

As will be described below in the examples, the present inventors have revealed that, in the cells including the dormant cancer stem cells in cancer tissue during chemotherapy, activation of YAP signaling after chemotherapy causes the cells to regrow, resulting in the relapse or recurrence of cancer.

Therefore, it can be determined that a test substance that inhibits activation of YAP signaling in the cells including cancer stem cells in cancer tissue after chemotherapy is a prophylactic or therapeutic agent against relapse or recurrence of cancer.

In the screening method of the present embodiment, the same as described above is applied to relapse or recurrence of cancer, chemotherapy, a dormant state, YAP signaling, a test substance, and the like. The dormant cancer stem cells are preferably LGR5-positive p27-positive cells.

The activation of YAP signaling can be evaluated by, for example, measuring pFAK, measuring nuclear translocation of YAP, and measuring the expression level of YAP target genes (YAP/TAZ/TEAD target genes).

The suppression of the activation of YAP signaling in the presence of a test substance may be, for example, a decrease in the expression level of pFAK in the presence of a test substance, a decrease in the amount of nuclear-translocated YAP, and a decrease in the expression level of YAP target genes, compared to the absence of a test substance. Examples of YAP target genes include LATS2, CTGF, and CYR61.

### (Third Embodiment)

In one embodiment, the present invention provides a method for screening a prophylactic or therapeutic agent against relapse or recurrence of cancer due to cells including dormant cancer stem cells in cancer tissue during chemotherapy, the method including: a step of incubating LGR5-positive p27-positive cancer stem cells dissociated into single cells in the presence of a test substance; and a step of evaluating the proliferation of the LGR5-positive p27-positive cancer stem cells, in which the fact that the proliferation of the LGR5-positive p27-positive cancer stem cells is inhibited compared to that in the absence of the test substance indicates that the test substance is a prophylactic or therapeutic agent against relapse or recurrence of cancer due to cells including dormant cancer stem cells in cancer tissue during chemotherapy.

As will be described below in the examples, the present inventors have revealed that the dormant cancer stem cells during chemotherapy are LGR5-positive p27-positive cancer stem cells. The present inventors have also revealed that COL17A1-mediated cell-extracellular substrate adhesion is necessary for maintenance of dormant LGR5-positive p27-positive cancer stem cells.

Dissociation of LGR5-positive p27-positive cancer stem cells into single cells disrupts the cell-extracellular substrate adhesion. This results in activation of FAK-YAP signaling (YAP signaling) in the LGR5-positive p27-positive cancer stem cells, thereby releasing the dormant state of the LGR5-positive p27-positive cancer stem cells.

Therefore, it can be determined that the test substance which inhibits the proliferation of the LGR5-positive p27-positive cancer stem cells dissociated into single cells is a prophylactic or therapeutic agent against relapse or recurrence of cancer.

The method for dissociating LGR5-positive p27-positive cancer stem cells into single cells is not particularly limited, and may be an enzymatic dissociation method, a physical dissociation method, or a combination of these methods. In the case of enzymatic dissociation, collagenase, dispase I, liberase, trypsin, TrypLE Express (Thermo Fisher Scientific Inc.), and the like can be used as enzymes. Physical dissociation can be carried out by mechanical shearing, for example, by pipetting.

In the screening method of the present embodiment, the same as described above is applied to relapse or recurrence of cancer, chemotherapy, a dormant state, YAP signaling, a test substance, and the like.

### (Fourth Embodiment)

In one embodiment, the present invention provides a method for screening a prophylactic or therapeutic agent against relapse or recurrence of cancer due to cells including dormant cancer stem cells in cancer tissue during chemotherapy, the method including: a step of incubating LGR5-positive p27-positive cancer stem cells dissociated into single cells in the presence of a test substance; and a step of evaluating activation of YAP signaling in the LGR5-positive p27-positive cancer stem cells, in which the fact that the activation of YAP signaling is inhibited compared to that in the absence of the test substance indicates that the test substance is a prophylactic or therapeutic agent against relapse or recurrence of cancer due to cells including dormant cancer stem cells in cancer tissue during chemotherapy.

The screening method of the fourth embodiment differs mainly from the screening method of the third embodiment in that it evaluates the activation of YAP signaling in the LGR5-positive p27-positive cancer stem cells.

As will be described below in the examples, the present inventors have revealed that, in the dormant LGR5-positive p27-positive cancer stem cells during chemotherapy, the activation of YAP signaling after chemotherapy causes the cells to regrow, resulting in the relapse or recurrence of cancer.

The present inventors have also revealed that COL17A1-mediated cell-extracellular substrate adhesion is necessary for maintenance of dormant LGR5-positive p27-positive cancer stem cells. Dissociation of LGRS-positive p27-positive cancer stem cells into single cells disrupts the cell-extracellular substrate adhesion. This results in activation of FAK-YAP signaling (YAP signaling) in the LGR5-positive p27-positive cancer stem cells, thereby releasing the dormant state of the LGR5-positive p27-positive cancer stem cells.

Therefore, it can be determined that the test substance which inhibits the activation of YAP signaling in the LGR5-positive p27-positive cancer stem cells dissociated into single cells is a prophylactic or therapeutic agent against relapse or recurrence of cancer.

In the screening method of the present embodiment, the same as described above is applied to relapse or recurrence of cancer, chemotherapy, a dormant state, YAP signaling, a test substance, dissociation into single cells, and the like.

The activation of YAP signaling can be evaluated in the same manner as in the second embodiment, for example, by measuring pFAK, measuring nuclear translocation of YAP, and measuring the expression level of YAP target genes (YAP/TAZ/TEAD target genes).

The suppression of the activation of YAP signaling in the presence of a test substance may be, for example, a decrease in the expression level of pFAK in the presence of a test substance, a decrease in the amount of nuclear-translocated YAP, and a decrease in the expression level of YAP target genes, compared to the absence of a test substance. Examples of YAP target genes include LATS2, CTGF, and CYR61.

### (Fifth Embodiment)

In one embodiment, the present invention provides a method for screening a prophylactic or therapeutic agent against relapse or recurrence of cancer due to cells including dormant cancer stem cells in cancer tissue during chemotherapy, the method including: a step of incubating LGR5-positive p27-positive cancer stem cells dissociated into single cells in the presence of a test substance; and a step of evaluating expression of COL17A1 in the LGR5-positive p27-positive cancer stem cells, in which the fact that the expression of COL17A1 is elevated compared to that in the absence of the test substance indicates that the test substance is a prophylactic or therapeutic agent against relapse or recurrence of cancer due to cells including dormant cancer stem cells in cancer tissue during chemotherapy.

As will be described below in the examples, the present inventors have revealed that COL17A1-mediated cell-extracellular substrate adhesion is necessary for maintenance of dormant LGR5-positive p27-positive cancer stem cells.

The present inventors have also revealed that expression of COL17A1 is lost in cancer cells on which a chemotherapeutic agent is allowed to act, and FAK-YAP signaling is activated.

Dissociation of LGR5-positive p27-positive cancer stem cells into single cells disrupts the cell-extracellular substrate adhesion. This results in activation of FAK-YAP signaling in the LGR5-positive p27-positive cancer stem cells, thereby releasing the dormant state of the LGR5-positive p27-positive cancer stem cells.

Therefore, it can be determined that the test substance which elevates the expression of COL17A1 in the LGR5-positive p27-positive cancer stem cells dissociated into single cells is a prophylactic or therapeutic agent against relapse or recurrence of cancer.

In the screening method of the present embodiment, the same as described above is applied to relapse or recurrence of cancer, chemotherapy, a dormant state, YAP signaling, a test substance, dissociation into single cells, and the like.

### (Sixth Embodiment)

In one embodiment, the present invention provides a method for screening a prophylactic or therapeutic agent against relapse or recurrence of cancer due to cells including dormant cancer stem cells in cancer tissue during chemotherapy, the method including: a step of allowing a chemotherapeutic agent to act on cancer organoids in the presence of a test substance; and a step of evaluating expression of COL17A1 in the cancer organoids, in which the fact that the expression of COL17A1 is elevated compared to that in the absence of the test substance indicates that the test substance is a prophylactic or therapeutic agent against relapse or recurrence of cancer due to cells including dormant cancer stem cells in cancer tissue during chemotherapy.

The screening method of the sixth embodiment differs mainly from the screening method of the fifth embodiment in that cell-extracellular substrate adhesion is disrupted by eliminating the expression of COL17A1 through the action of a chemotherapeutic agent on cancer organoids instead of disrupting cell-extracellular substrate adhesion by dissociating cancer organoids into single cells.

As described above, the present inventors have revealed that, in cancer cells on which a chemotherapeutic agent is allowed to act, the expression of COL17A1 is lost, and FAK-YAP signaling is activated, thereby releasing the dormant state of the LGR5-positive p27-positive cancer stem cells.

Therefore, it can be determined that the test substance which elevates the expression of COL17A1 reduced by the action of the chemotherapeutic agent is a prophylactic or therapeutic agent against relapse or recurrence of cancer.

In the screening method of the present embodiment, the same as described above is applied to a test substance, relapse or recurrence of cancer, chemotherapy, a dormant state, YAP signaling, a test substance, and the like.

### [Other embodiments]

In one embodiment, the present invention provides a preventive or therapeutic method for relapse or recurrence of cancer due to dormant cancer stem cells during chemotherapy, the method including a step of administering an effective amount of a YAP inhibitor to a post-chemotherapy cancer patient.

In one embodiment, the present invention provides a YAP inhibitor for use in the prevention or treatment of relapse or recurrence of cancer due to dormant cancer stem cells in a post-chemotherapy cancer patient during chemotherapy.

In one embodiment, the present invention provides use of a YAP inhibitor for producing a prophylactic or therapeutic agent against relapse or recurrence of cancer due to dormant cancer stem cells during chemotherapy.

In each of these embodiments, the same as described above is applied to relapse or recurrence of cancer, chemotherapy, a dormant state, a YAP inhibitor, and the like. Examples

Examples will be shown below to describe the present invention in more detail, but the present invention is not limited to the following examples.

### [Experimental Example 1]

### (Establishment of in vivo tracing technology for cancer stem cells)

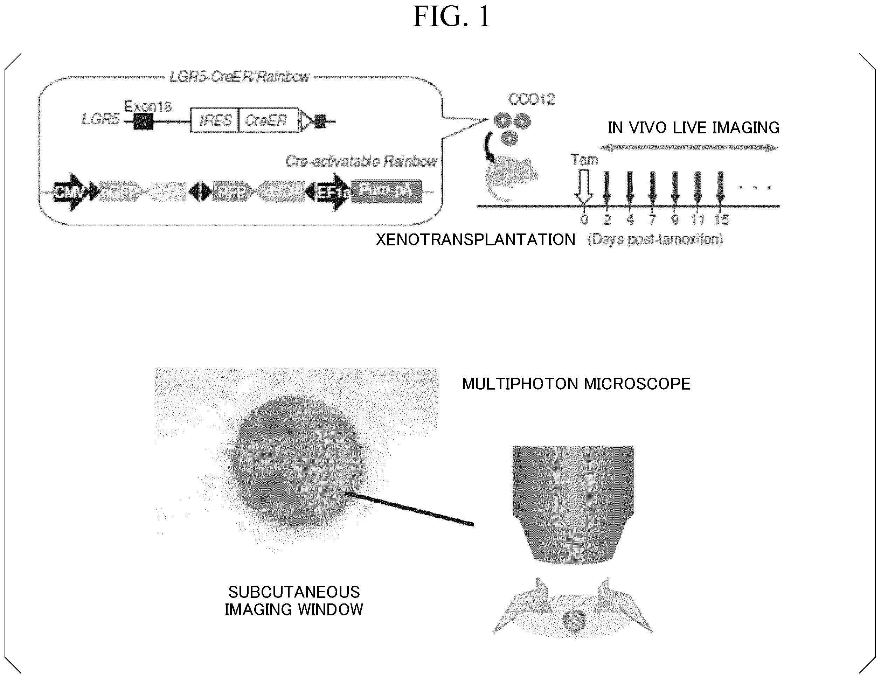

Technology to observe human cancer stem cell over time at a single cell level through in vivo imaging was established. FIG. 1 is a schematic view illustrating this experimental system. First, human colorectal cancer-derived organoids including LGR5-positive cancer stem cells labeled with a cell lineage marker were prepared. For the cell lineage marker, Shimokawa M., et al., Visualization and targeting of LGR5+ human colon cancer stem cells, Nature, 545 (7653), 187-192, 2017 may be referred to, for example.

These organoids ubiquitously express nuclear GFP. In addition, when subjected to tamoxifen treatment, random recombination occurs in a plurality of loxP sequences present in a rainbow cassette. As a result, LGR5-positive cells are probabilistically labeled with yellow fluorescent protein (YFP), red fluorescent protein (RFP), or cyan fluorescent protein (CFP).

Once cells have undergone recombination expressing one of the fluorescent proteins, and their offspring continue to express the same fluorescent protein. Therefore, cells derived from a single cell can be tracked and analyzed. Hereinafter, organoids labeled with the above-described cell lineage marker may be referred to as "LGR5-CreER/Rainbow organoids."

The LGR5-CreER/Rainbow organoids are xenotransplanted under the skin of immunodeficient NOG mice. Subsequently, in vivo imaging of the subcutaneously transplanted organoids was carried out using an imaging window device. Using this system, live tumor cells could be observed at single-cell resolution for 30 days or longer using a multiphoton microscope.

### [Experimental Example 2]

### (In vivo tracing of cancer stem cells)

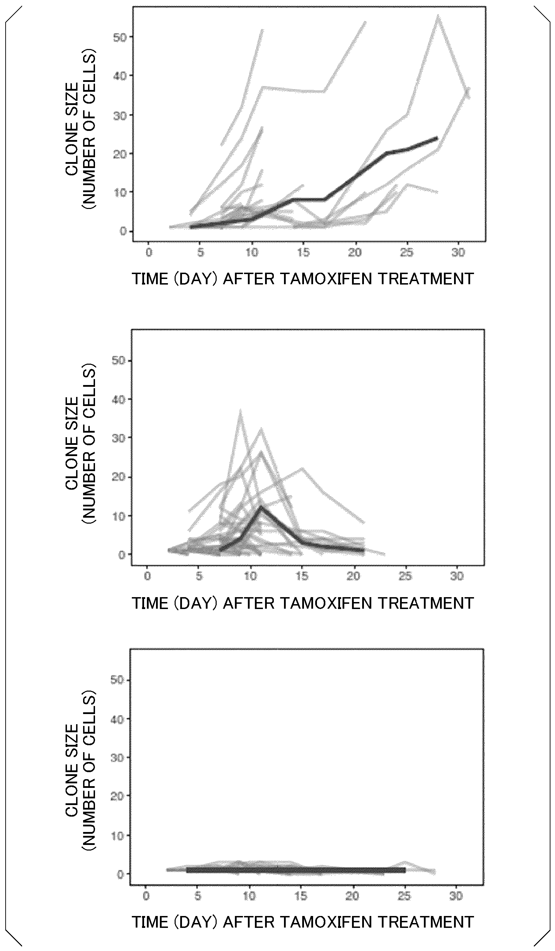

It was revealed that three patterns of clones are present in the xenotransplanted mice of Experimental Example 1 by tracking labeled LGR5-positive clones. FIG. 2 is a graph showing results of measuring cell proliferation of each cell of the three patterns.

The upper row in FIG. 2 shows a pattern of continuously expanding clones (n=23). Clones consisting of four or more cells that had no reduction in size of 50% or more and were traceable for six days or longer were defined as continuously expanding clones. The continuously expanding clones showed stable proliferation and were consistent with typical cancer stem cell models. About 24% of the clones were continuously expanding clones.

The middle row in FIG. 2 shows a pattern of diminishing clones (n=51). Clones which consist of three or less cells and declined within six days or clones which consist of four or more cells and reduced in size by 50% or more were defined as diminishing clones. The diminishing clones initially expanded but then declined. About 54% of the clones were diminishing clones.

The lower row in FIG. 2 shows a pattern of dormant clones (n=20). Clones that consist of three or less cells and could be tracked for six days or longer were defined as dormant clones. Dormant clones rarely divided and maintained a minute clone size, indicating that they were dormant cancer cells. About 21% of the clones were dormant clones.

As described above, it was possible to faithfully visualize diverse dynamics of the LGR5-positive colorectal cancer cells in vivo at single-cell resolution.

### [Experimental Example 3]

### (Identification of markers of dormant clones)

Ki67 protein is a cell proliferation potential marker. Ki67 is known to be present in all cell cycles (G1, S, G2, and M phases) of proliferating cells, but not in the resting phase (G0 phase), where cell proliferation is dormant.

To identify markers of dormant clones, that is, LGR5-positive Ki67-negative human colorectal cancer stem cells, the expression patterns of cell cycle inhibitory proteins, p21, p57, and p27, expressed in LGR5-positive colorectal cancer stem cells were screened. As a result, it was revealed that the LGR5-positive Ki67-negative colorectal cancer stem cells express mainly p27, with the p27 expressing indicating a slowly dividing population or a resting state population.

Analysis of four different human colorectal cancer organoid strains (CCO7, CCO20, CCO25, and CCO32) confirmed the presence of LGR5-positive p27-positive cells in all of these organoids. In addition, DNA/RNA content analysis confirmed that the LGR5-positive p27-positive cells were mainly in the G0 phase.

The above results revealed that the LGR5-positive colorectal cancer dormant clones expressed p27.

### [Experimental Example 4]

### (LGR5-Positive p27-positive cells were resistant to chemotherapeutic agent)

In vitro live imaging was used to track proliferation dynamics of LGR5-positive p27-positive cells and LGR5-positive p27-negative cells in human colorectal cancer organoids.

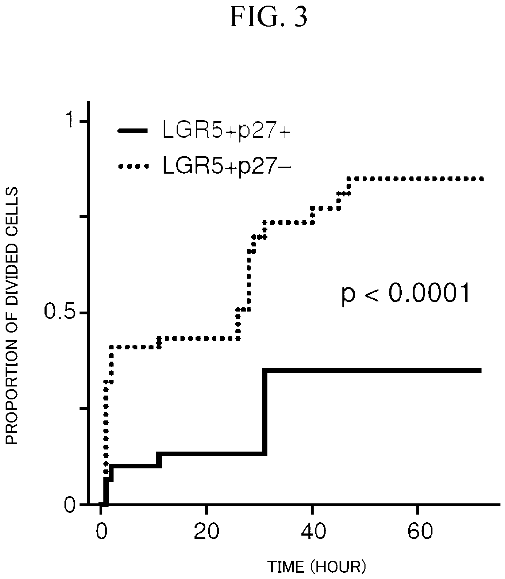

FIG. 3 is a graph showing results of measuring the proportion of divided cells over time by in vitro live imaging. As a result, more than 80% of the LGR5-positive p27-negative cells divided and proliferated, whereas more than two-thirds of the LGR5-positive p27-negative cells remained single cells.

The human colorectal cancer organoids were treated with camptothecin (CPT) which is a topoisomerase inhibitor (50 nM). Subsequently, the LGR5-positive p27-positive cells, the LGR5-positive p27-negative cells, LGR5-negative p27-positive cells, and LGR5-negative p27-negative cells were sorted and collected with a cell sorter, and 1,000 cells of each type were cultured.

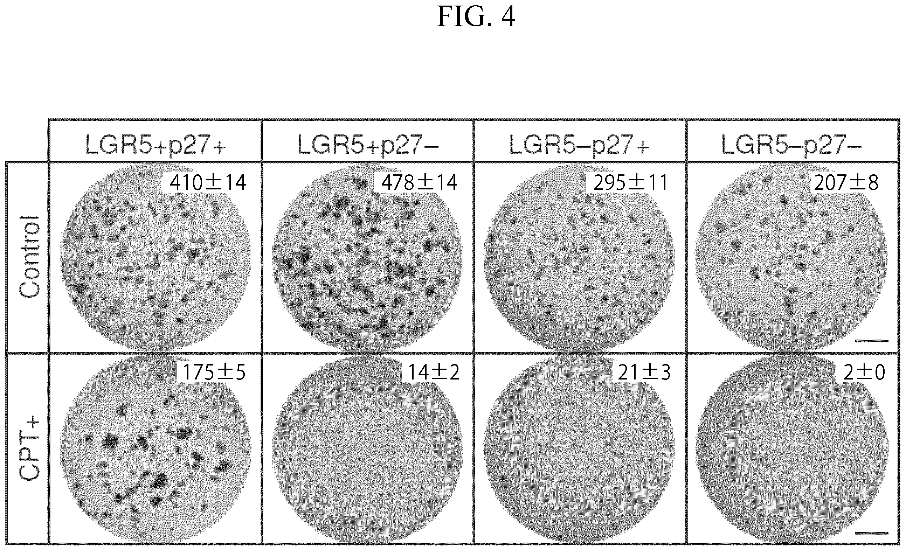

FIG. 4 shows photographs showing each of the cells on day 13 after sorting. The numerical values in FIG. 4 show the number of colonies (average value ± standard error). The scale bar is 1 mm. As a result, the LGR5-positive p27-positive cells after camptothecin treatment showed colony formation, but the LGR5-positive p27-negative cells after camptothecin treatment showed no colony formation. The results indicate that the LGR5-positive p27-positive cells are resistant to the chemotherapeutic agent.

### [Experimental Example 5]

### (In vivo imaging of LGR5-positive p27-positive cells)

Whether or not the LGR5-positive p27-positive cells resistant to the chemotherapeutic agent contribute to relapse or recurrence of tumors after chemotherapy was studied in vivo. Specifically, irinotecan was administered to mice xenotransplanted with human colorectal cancer organoids, followed by in vivo imaging and genetic lineage-tracing of the LGR5-positive p27-positive cells and the LGR5-positive p27-negative cells over time.

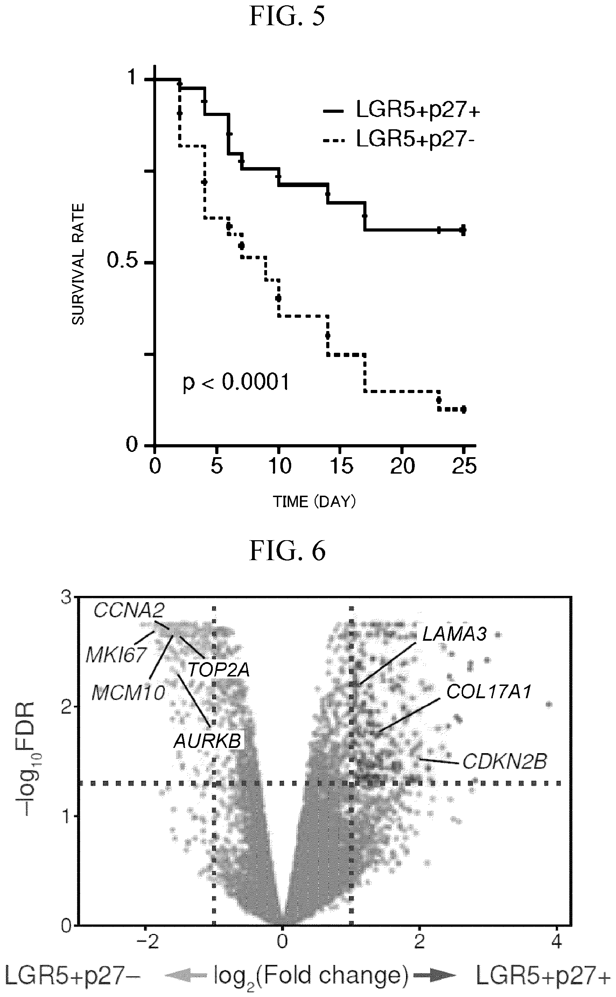

FIG. 5 is a graph showing results obtained by measuring the survival rate of LGR5-positive p27-positive cells and LGR5-positive p27-negative cells after administration of irinotecan. The results confirmed that the LGR5-positive p27-positive cells tolerated chemotherapy and remained. On the other hand, the LGR5-positive p27-negative cells disappeared during an observation period.

These results suggest that the LGR5-positive p27-positive cells function as dormant cancer stem cells resistant to chemotherapeutic agents and contribute to cancer relapse or recurrence of cancer after chemotherapy.

### [Experimental Example 6]

### (Cell-extracellular substrate interface regulated dormancy of cancer stem cells)

The present inventors have found that when LGR5-positive p27-positive cells were dissociates into single cells and subcultured, expression of p27 was lost and caused cell division.

Upon dissociation into single cells, the separation was carried out from the extracellular substrate and adjacent cells. Therefore, whether either cell-extracellular substrate adhesion or cell-cell adhesion is required for maintenance of the LGR5-positive p27-positive cells was studied. Specifically, two types of cells including the LGR5-positive p27-positive single cells and LGR5-positive p27-positive cells that maintain cell-cell adhesion were sorted, collected, and observed. As a result, both types of cells similarly underwent cell division.

The results suggest that cell-extracellular substrate adhesion, rather than cell-cell adhesion, contributes to maintaining the dormant state.

FIG. 6 is a volcano plot showing results obtained by comparing the LGR5-positive p27-positive cells with the LGR5-positive p27-negative cells.

The present inventors focused on COL17A1 among genes whose expression was specifically elevated in the LGR5-positive p27-positive cells. COL17A1 is a structural component of hemidesmosome that enhances adhesion between epithelial cells and basement membranes or Matrigel.

### [Experimental Example 7]

### (COL17A1 maintained dormancy of cancer stem cells)

To investigate the functional role of COL17A1 in the dormancy of cancer stem cells, human colorectal cancer organoids (CC07 strain and CCO32 strain) in which COL17A1 was knocked out with CRISPR-Cas9 were produced.

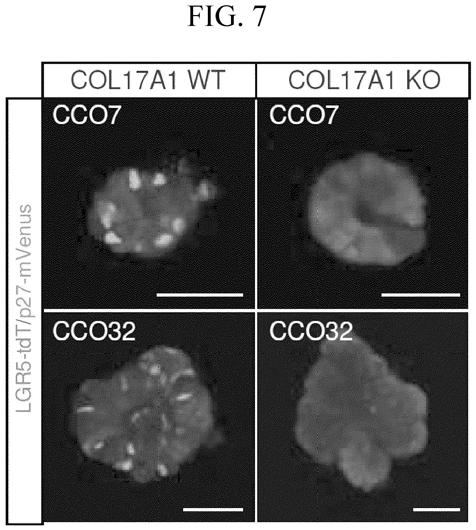

FIG. 7 shows fluorescence micrographs showing expression of LGR5 and p27 in human colorectal cancer organoids expressing COL17A1 and human colorectal cancer organoids in which COL17A1 is knocked out. In FIG. 7, "COL17A1 WT" indicates a human colorectal cancer organoid expressing COL17A1, and "COL17A1 KO" indicates a human colorectal cancer organoid in which COL17A1 is knocked out. The scale bar is 100 µm.

Interestingly, the results revealed that, in COL17A1 KO organoids, the expression of LGR5 was maintained, but dormant LGR5-positive p27-positive cells were lost.

In addition, it was also confirmed that rescue of the expression of COL17A1 restored p27-positive cells by doxycycline (Dox)-induced COL17A1 overexpression.

These results suggest that COL17A1 regulates dormancy of cancer stem cells.

### [Experimental Example 8]

### (Study of chemotherapy sensitivity of COL17A1 KO organoids)

The chemotherapy sensitivity of COL17A1 KO human colorectal cancer organoids was studied. The results revealed that the COL17A1 KO organoids were more sensitive to camptothecin compared to COL17A1 WT organoids, and COL17A1 single allele KO (hetero KO) organoids. It was suggested that, in the COL17A1 KO organoids, the lose of LGR5-positive p27-positive cells made the COL17A1 KO organoids vulnerable to chemotherapy.

Subsequently, irinotecan was administered to mice xenotransplanted with COL17A1 KO human colorectal cancer organoids, and the tumor size was measured over time.

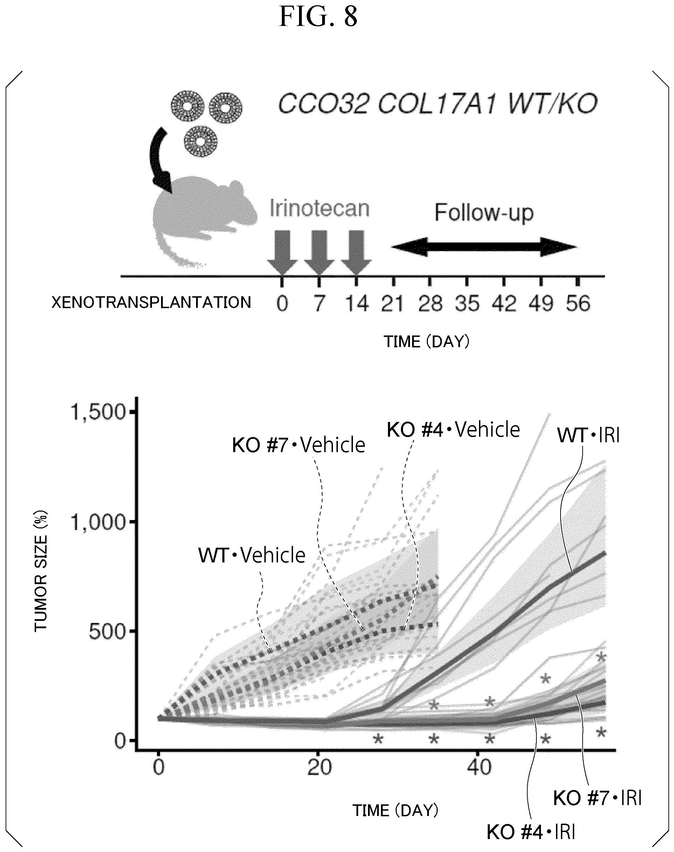

The upper row in FIG. 8 is a schematic view showing a schedule of this experiment. The lower row in FIG. 8 is a graph showing change in tumor size over time. In FIG. 8, the term "Vehicle" indicates a control to which a solvent is administered, and the term "IRI" indicates that irinotecan is administered. In addition, "*" indicates a significant difference at p < 0.05.

The results revealed that tumor regeneration after administration of irinotecan was more inhibited in COL17A1 KO xenografts than in COL17A1 WT xenografts. The results were thought to reflect depletion of dormant cancer cells with the ability to regrow after chemotherapy in the COL17A1 KO xenografts.

In addition, the similar experiment was also carried out using colorectal cancer organoids derived from another patient, and as a result, the same results as those in this experimental example were obtained.

The above results suggested that COL17A1 prevented the proliferation of dormant cancer cells, and the lose thereof increased the effect of chemotherapy.

### [Experimental Example 9]

### (Dormancy of COL17A-FAK-YAP pathway regulates dormancy of cancer stem cells)

It was analyzed how COL17A1 regulates the dormancy in human colorectal cancer organoids. A probability that lose of COL17A1 could fluctuate hemidesmosome adhesion and focal adhesion signals could be alternatively activated was considered. Expression of vinculin and phosphorylated FAK^{Y397} (pFAK) which are constituent molecules of focal adhesion signals in COL17A1 KO organoids and activation of YAP signaling (nuclear translocation of YAP) downstream of FAK were analyzed.

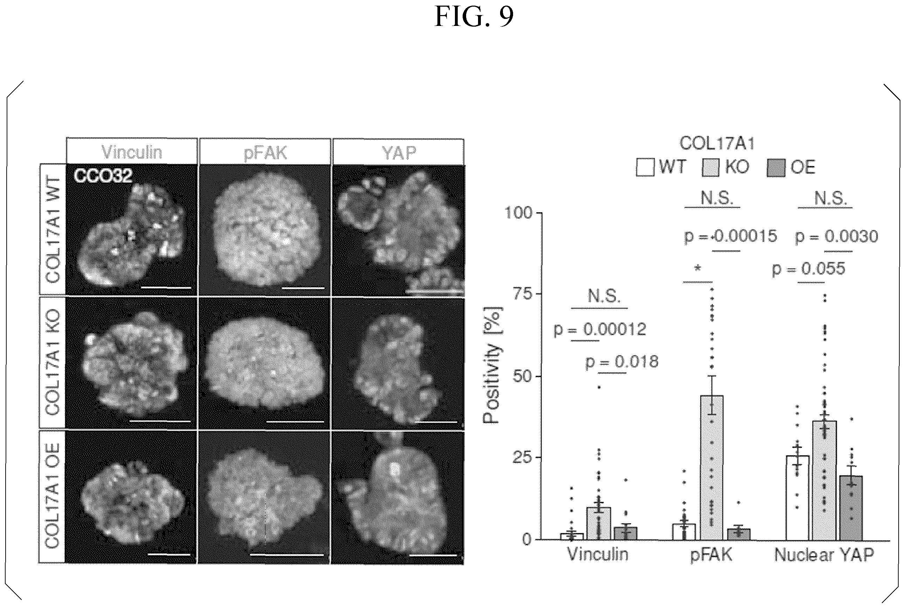

The left side of FIG. 9 shows representative confocal micrographs showing results of analysis of the expression of vinculin and pFAK and the activation of YAP signaling. The scale bar is 100 µm. In addition, the right side of FIG. 9 is a graph showing results of measuring the expression of vinculin and pFAK and the activation of YAP signaling based on the photographs on the left side of FIG. 9. In the right side of FIG. 9, "N.S." indicates no significant difference.

The results confirmed that expression of both vinculin and pFAK was elevated in the COL17A1 KO organoids, suggesting the activation of focal adhesion signals. In addition, the activation of downstream YAP signaling was recognized.

Furthermore, the effect of the elevated expression of vinculin and pFAK was counteracted in COL17A1 OE organoids which were forced to express COL17A1 in the COL17A1 KO organoids.

As a result of gene set concentration analysis, YAP target genes were more concentrated in the COL17A1 KO organoids than in the COL17A1 OE organoids. These results suggested that the deficiency of COL17A1 induces the activation of FAK-YAP.

Subsequently, the YAP state of the human colorectal cancer organoids was analyzed to investigate whether the activation of FAK-YAP affects the dormant state of LGR5-positive p27-positive cells.

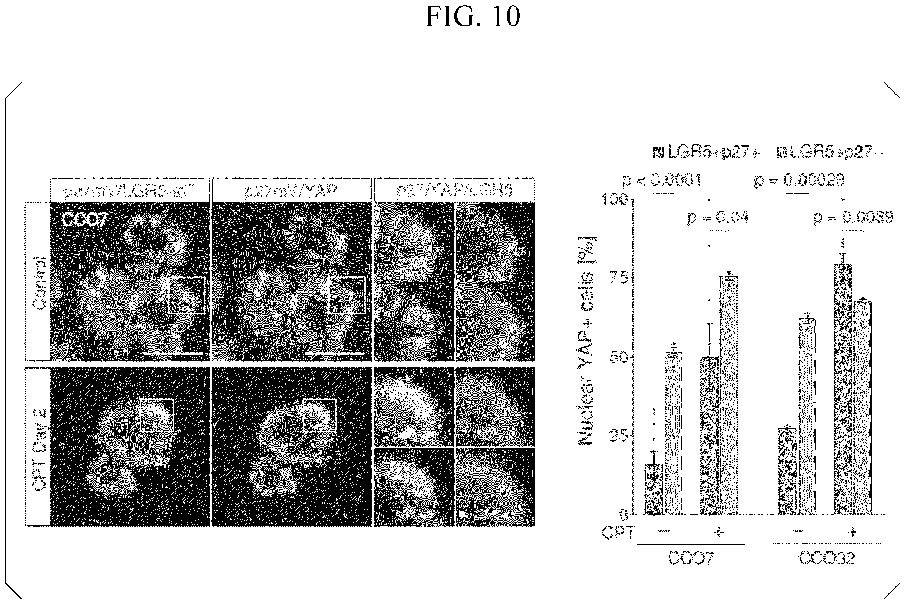

The left side of FIG. 10 shows representative confocal micrographs showing results of analysis of nuclear translocation of LGRS, p27, and YAP in human colorectal cancer organoids (CCO7 strain and CCO32 strain) subjected to camptothecin treatment and human colorectal cancer organoids which have not been subjected to camptothecin treatment. The scale bar is 100 µm. The right side of FIG. 10 is a graph showing results of measuring the proportion of cells containing YAP that has undergone nuclear translocation based on the photographs on the left side of FIG. 10.

As a result, the level of nuclear localization of YAP was lower in the LGR5-positive p27-positive cells than in the LGR5-positive p27-negative cells, suggesting that YAP signaling negatively regulates the dormancy of the LGR5-positive p27-positive cells. In addition, it was suggested that the camptothecin treatment activates YAP signaling in the LGR5-positive p27-positive cells.

### [Experimental Example 10]

### (Study of YAP signaling inhibitor)

A YAP signaling inhibitor was allowed to act on COL17A1 KO organoids to study an effect on dormancy of cancer stem cells. As a YAP signaling inhibitor, MYF-01-37 (CAS No. 2416417-65-5), a low-molecular-weight TEAD inhibitor (TEADi) that disrupts YAP/TAZ-TEAD protein-protein interactions, was used (20 µM, 2 days).

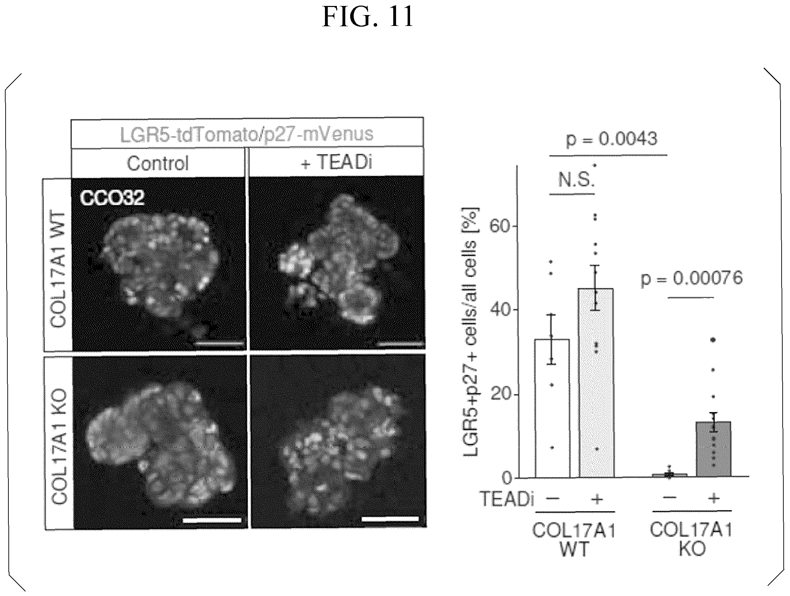

The left side of FIG. 11 shows representative confocal micrographs showing results of analysis of expression of LGR5 and p27 in human colorectal cancer organoids (COL17A1 KO or COL17A1 WT) treated with the YAP signaling inhibitor. The scale bar is 100 µm. The right side of FIG. 11 is a graph showing results of measuring the proportion of LGRS-positive p27-positive cells based on the photographs on the left side of FIG. 11.

The results revealed that blocking of YAP signaling in the COL17A1 KO organoids led to reappearance of p27-positive cells.

The results suggest that the expression of COL17A1 inhibited the activation of FAK-YAP signaling, thereby inhibiting cell cycle reentry of the p27-positive cells.

### [Experimental Example 11]

### (Chemotherapy inhibited expression of COL17A1)

Live imaging confirmed that LGR5-positive p27-positive cells remained resistant to chemotherapeutic agents and were reactivated after chemotherapy. However, it was unclear how chemotherapy would release the dormant state of cancer stem cells.

Therefore, whether or not the COL17A1-FAK-YAP pathway was involved in the release of the dormant state of cancer stem cells after chemotherapy.

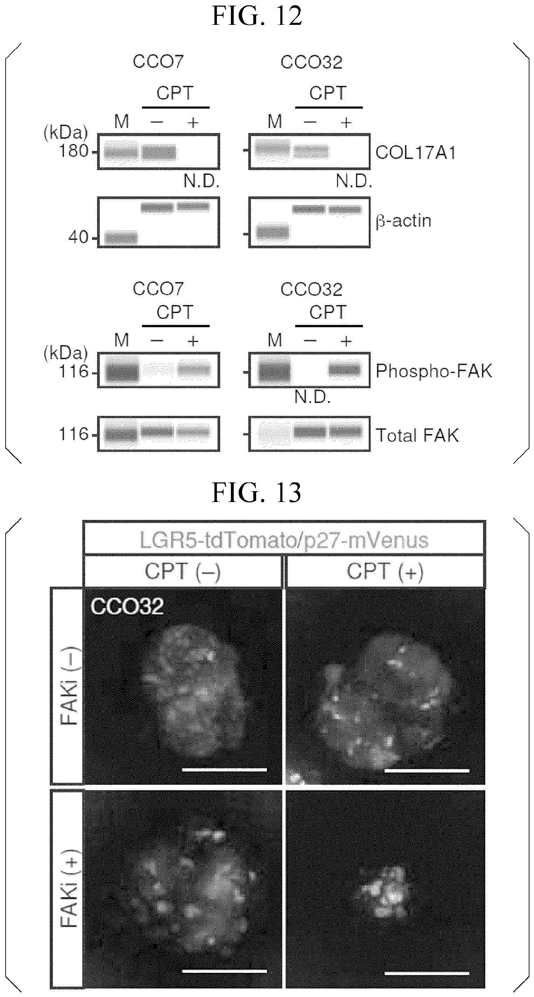

FIG. 12 shows images showing results of a capillary-based immunoassay for detection of COL17A1, pFAK, and total FAK in camptothecin-treated (50 nM) human colorectal cancer organoids (CCO7 strain and CCO32 strain).

The results revealed that the expression of COL17A1 was deleted and phosphorylation of FAK occurred in the camptothecin-treated human colorectal cancer organoids. The results indicate that the COL17A1-FAK-YAP pathway was involved in the release of the dormant state of cancer stem cells after chemotherapy.

To further clarify the role of FAK-YAP signaling in the release of the dormant state of cancer stem cells after chemotherapy, FAK-YAP signaling was altered during and after camptothecin treatment.

FIG. 13 shows representative confocal micrographs showing results of camptothecin treatment of human colorectal cancer organoids (CCO32 strain) in the presence or absence of a FAK inhibitor (10 nM, 24 hours) and analysis of the expression of LGR5 and p27. The scale bar is 100 µm. As a FAK inhibitor, PF573228 (CAS No. 869288-64-2) was used (10 µm). In FIG. 13, "FAKi" indicates a FAK inhibitor.

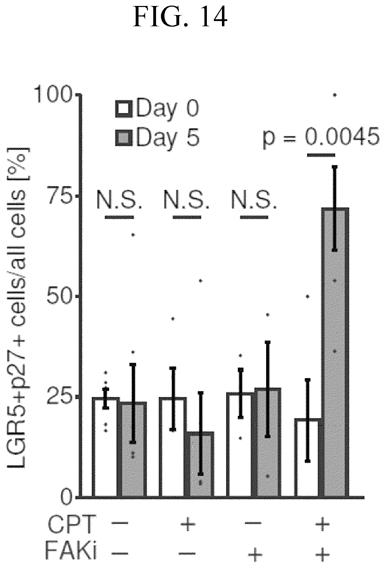

FIG. 14 is a graph showing results of measuring the proportion of the LGR5-positive p27-positive cells when the human colorectal cancer organoids are treated or not treated with camptothecin for 5 days in the presence or absence of a FAK inhibitor. In FIG. 14, "N.S." indicates no significant difference.

The results revealed that co-treatment of camptothecin with a FAK inhibitor inhibited cell cycle reentry of the LGR5-positive p27-positive cells after camptothecin exposure. These results suggested that inhibition of FAK inhibits the release of the dormant state of cancer stem cells after chemotherapy, preventing relapse or recurrence of cancer after chemotherapy.

### [Experimental Example 12]

### (Study 1 of YAP inhibitor)

Whether or not YAP inhibition could counteract tumor regrowth after chemotherapy by preventing the dormant state of the LGR5-positive p27-positive cells was studied.

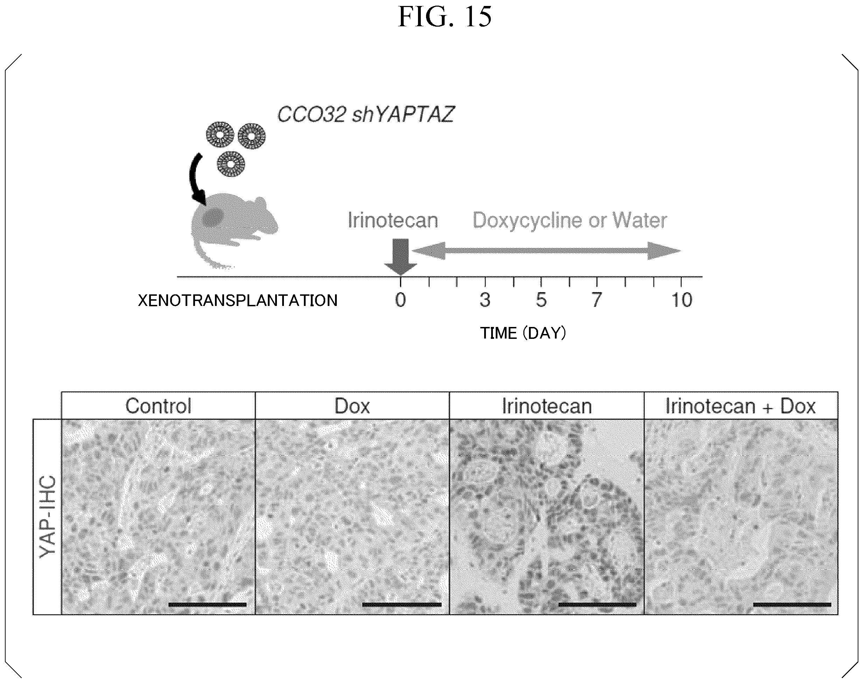

The upper row in FIG. 15 is a schematic view showing a schedule of this experiment. Doxycycline-inducible YAP/TAZ-knockdown human colorectal cancer organoids were produced and xenotransplanted into the subcutis of immunodeficient NOG mice. Thereafter, irinotecan was administered.

The lower row in FIG. 15 shows micrographs showing results of immunostaining a graft in each group after three days from administration of irinotecan. The scale bar is 100 µm. In the lower row in FIG. 15, "Control" indicates a result of a group into which neither irinotecan nor doxycycline has been administered, "Dox" indicates a result of a group into which only doxycycline was administered, "Irinotecan" indicates a result of a group into which only irinotecan was administered, and "Irinotecan + Dox" indicates a result of a group into which irinotecan and doxycycline were administered.

The results confirmed that administration of irinotecan resulted in nuclear translocation of YAP in vivo, but showed that the nuclear translocation of YAP was inhibited in the group into which irinotecan was administered together with doxycycline to knock down YAP/TAZ.

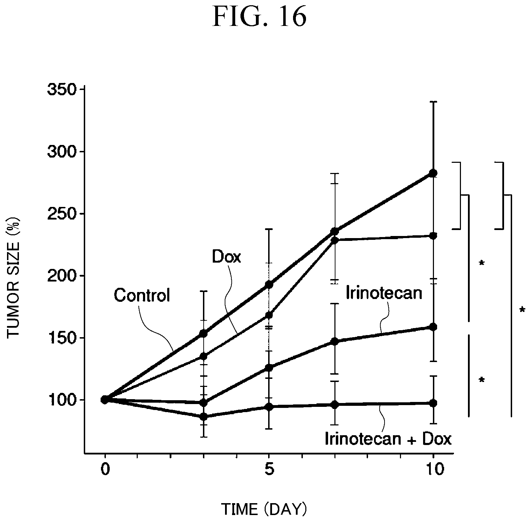

FIG. 16 is a group showing change in tumor size in mice of each group over time. As a result, in FIG. 16, "Control," "Dox," "Irinotecan," and "Irinotecan + Dox" show the same meaning as in the lower row in FIG. 15. In addition, "*" indicates a significant difference at p < 0.05.

The results revealed that, although the therapeutic effect with irinotecan was transient, the antitumor effect of irinotecan and the maintenance of the dormant state of cancer stem cells were enhanced in the group into which irinotecan was administered together with doxycycline to knock down YAP/TAZ.

### [Experimental Example 13]

### (Study 2 of YAP inhibitor)

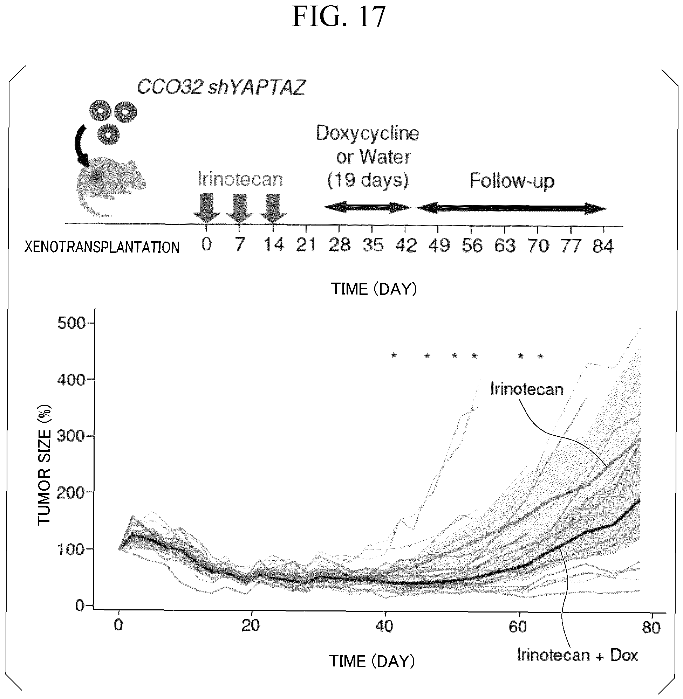

How the inhibition of YAP signaling affects tumor regrowth after chemotherapy was studied. The upper row in FIG. 17 is a schematic view showing a schedule of this experiment. Doxycycline-inducible YAP/TAZ-knockdown human colorectal cancer organoids were produced and xenotransplanted into the subcutis of immunodeficient NOG mice. Thereafter, irinotecan was administered once a week for three times, followed by administering doxycycline over a long period of time to inhibit YAP signaling. Thereafter, administration of doxycycline was discontinued.

The lower row in FIG. 17 is a group showing change in tumor size in mice of each group over time. In the lower row in FIG. 17, "Irinotecan" indicates a result of the group into which only irinotecan was administered, and "Irinotecan + Dox" indicates a result of the group into which irinotecan and doxycycline were administered.

The results showed a transient therapeutic effect in the group into which doxycycline was not administered after administration of irinotecan, and cancer regrew around day 40. In addition, the tumor size was reduced to a minimum by administration of doxycycline after administration of irinotecan. Furthermore, tumor regrowth was recognized when administration of doxycycline was discontinued.

### [Experimental Example 14]

### (Study 3 of YAP inhibitor)

It was studied that the application of a low-molecular-weight TEAD inhibitor (TEADi) in vivo as a drug supplementally used for chemotherapy with a view to clinical application of YAP targeting therapy.

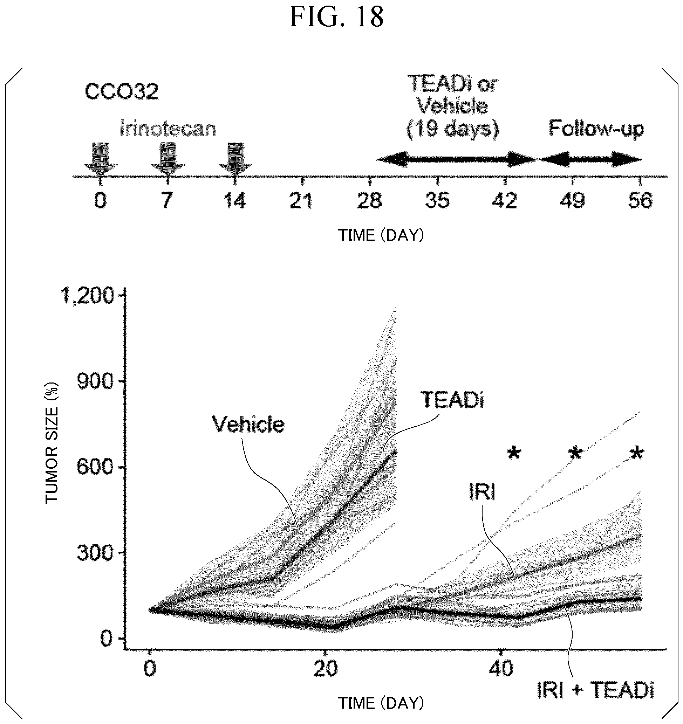

The upper row in FIG. 18 is a schematic view showing a schedule of this experiment. Human colorectal cancer organoids (CCO32 strain) were xenotransplanted into the subcutis of immunodeficient NOG mice. Thereafter, irinotecan was administered once a week for three times, followed by administering a TEAD inhibitor over a long period of time to inhibit YAP signaling. Thereafter, administration of the TEAD inhibitor was discontinued. As a TEAD inhibitor, MYF-01-37 (CAS No. 2416417-65-5) was used.

The lower row in FIG. 18 is a group showing change in tumor size in mice of each group over time. In the lower row in FIG. 18, "Vehicle" indicates a result of a group into which only a solvent was administered, "TEADi" indicates a result of a group into which only a TEAD inhibitor was administered, "IRI" indicates a result of a group into which only irinotecan was administered, and "IRI + TEADi" indicates a result of a group into which irinotecan and a TEAD inhibitor were administered.

As a result, the TEAD inhibitor alone did not show any tumor inhibition effect, but the tumor size was reduced to a minimum by administration of the TEAD inhibitor after administration of irinotecan. In addition, a transient therapeutic effect was shown in the group into which the TEAD inhibitor was not administered after administration of irinotecan, and cancer regrew around day 40.

### [Proof of concept]

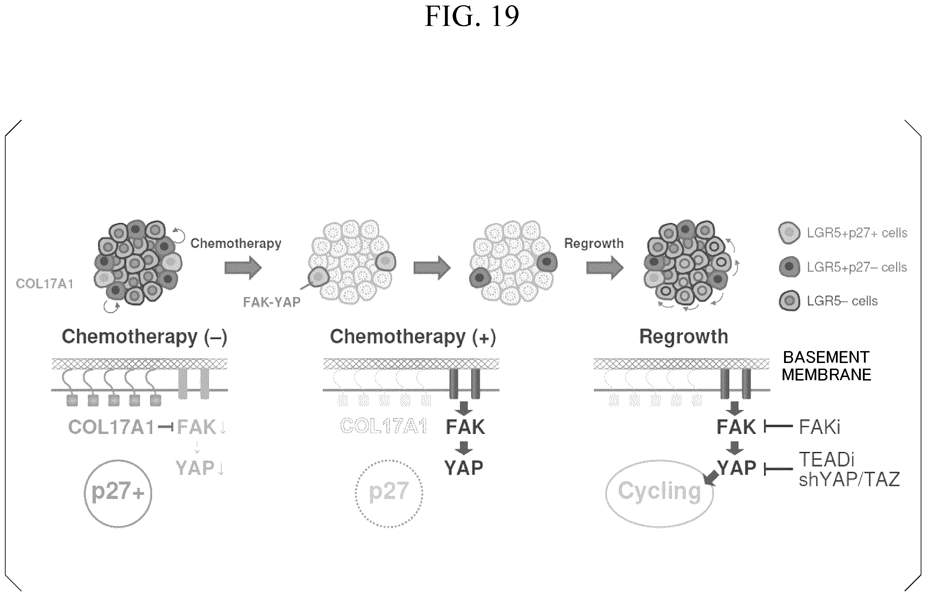

The upper row in FIG. 19 is a schematic view showing a mechanism by which the dormant state of the dormant LGR5-positive p27-positive cancer stem cells is released and a tumor regrows, as revealed from the above-described experimental results. Chemotherapy kills cancer cells, but the LGR5-positive p27-positive cancer stem cells survive. In the LGR5-positive p27-positive cancer stem cells that survived after chemotherapy, the FAK-YAP signaling is activated, resulting in negative p27 expression and regrowth.

The lower row in FIG. 19 is a schematic view illustrating states of maintenance of the dormant state of cancer stem cells using COL17A1, activation of FAK-YAP signaling after chemotherapy, and release of the dormant state.

### INDUSTRIAL APPLICABILITY

According to the present invention, it is possible to provide a prophylactic or therapeutic agent against relapse or recurrence of cancer after chemotherapy, and a screening method therefor.

## Claims

1. A prophylactic or therapeutic agent against relapse or recurrence of cancer due to cells including dormant cancer stem cells in cancer tissue during chemotherapy, the prophylactic or therapeutic agent comprising:
a YAP inhibitor as an active component.

2. The prophylactic or therapeutic agent according to claim 1,
wherein the dormant cancer stem cells during chemotherapy are LGR5-positive p27-positive cancer stem cells.

3. The prophylactic or therapeutic agent according to claim 1 or 2,
wherein the relapse or recurrence of cancer is relapse or recurrence of cancer after chemotherapy.

4. The prophylactic or therapeutic agent according to any one of claims 1 to 3,
wherein the YAP inhibitor is an inhibitor of YAP signaling activation.

5. A pharmaceutical composition for preventing or treating relapse or
recurrence of cancer due to cells including dormant cancer stem cells in cancer tissue during chemotherapy, the composition comprising:
the prophylactic or therapeutic agent according to any one of claims 1 to 4; and
a pharmaceutically acceptable carrier.

6. A method for screening a prophylactic or therapeutic agent against relapse or recurrence of cancer due to cells including dormant cancer stem cells in cancer tissue during chemotherapy, the method comprising:
a step (a) of allowing a chemotherapeutic agent to act on cancer organoids, resulting in death of most cells of the cancer organoids;
a step (b) of further incubating the cancer organoids, resulting in start of proliferation of cells including dormant cancer stem cells present in the cancer organoids; and
a step (c) of evaluating the proliferation of the cells including the dormant cancer stem cells,
wherein the step (a) or (b) is carried out in the presence of a test substance, and the fact that the proliferation of the cells including the dormant cancer stem cells is inhibited compared to that in the absence of the test substance indicates that the test substance is a prophylactic or therapeutic agent against relapse or recurrence of cancer due to cells including dormant cancer stem cells in cancer tissue during chemotherapy.

7. A method for screening a prophylactic or therapeutic agent against relapse or recurrence of cancer due to cells including dormant cancer stem cells in cancer tissue during chemotherapy, the method comprising:
a step (a) of allowing a chemotherapeutic agent to act on cancer organoids, resulting in death of most cells of the cancer organoids;
a step (b) of further incubating the cancer organoids, resulting in start of proliferation of cells including dormant cancer stem cells present in the cancer organoids; and
a step (c) of evaluating activation of YAP signaling in the cells including the dormant cancer stem cells,
wherein the step (a) or (b) is carried out in the presence of a test substance, and the fact that the activation of YAP signaling is inhibited compared to that in the absence of the test substance indicates that the test substance is a prophylactic or therapeutic agent against relapse or recurrence of cancer due to cells including dormant cancer stem cells in cancer tissue during chemotherapy.

8. A method for screening a prophylactic or therapeutic agent against relapse or recurrence of cancer due to cells including dormant cancer stem cells in cancer tissue during chemotherapy, the method comprising:
a step of incubating LGR5-positive p27-positive cancer stem cells dissociated into single cells in the presence of a test substance; and
a step of evaluating the proliferation of the LGR5-positive p27-positive cancer stem cells,
wherein the fact that the proliferation of the LGR5-positive p27-positive cancer stem cells is inhibited compared to that in the absence of the test substance indicates that the test substance is a prophylactic or therapeutic agent against relapse or recurrence of cancer due to cells including dormant cancer stem cells in cancer tissue during chemotherapy.

9. A method for screening a prophylactic or therapeutic agent against relapse or recurrence of cancer due to cells including dormant cancer stem cells in cancer tissue during chemotherapy, the method comprising:
a step of incubating LGR5-positive p27-positive cancer stem cells dissociated into single cells in the presence of a test substance; and
a step of evaluating activation of YAP signaling in the LGR5-positive p27-positive cancer stem cells,
wherein the fact that the activation of YAP signaling is inhibited compared to that in the absence of the test substance indicates that the test substance is a prophylactic or therapeutic agent against relapse or recurrence of cancer due to cells including dormant cancer stem cells in cancer tissue during chemotherapy.

10. A method for screening a prophylactic or therapeutic agent against relapse or recurrence of cancer due to cells including dormant cancer stem cells in cancer tissue during chemotherapy, the method comprising:
a step of incubating LGR5-positive p27-positive cancer stem cells dissociated into single cells in the presence of a test substance; and
a step of evaluating expression of COL17A1 in the LGR5-positive p27-positive cancer stem cells,
wherein the fact that the expression of COL17A1 is elevated compared to that in the absence of the test substance indicates that the test substance is a prophylactic or therapeutic agent against relapse or recurrence of cancer due to cells including dormant cancer stem cells in cancer tissue during chemotherapy.

11. A method for screening a prophylactic or therapeutic agent against relapse or recurrence of cancer due to cells including dormant cancer stem cells in cancer tissue during chemotherapy, the method comprising:
a step of allowing a chemotherapeutic agent to act on cancer organoids in the presence of a test substance; and
a step of evaluating expression of COL17A1 in the cancer organoids,
wherein the fact that the expression of COL17A1 is elevated compared to that in the absence of the test substance indicates that the test substance is a prophylactic or therapeutic agent against relapse or recurrence of cancer due to cells including dormant cancer stem cells in cancer tissue during chemotherapy.
